# EUROPÄISCHE PATENTSCHRIFT

(11) **EP 1 181 922 B1**
(45) Veröffentlichungstag und Bekanntmachung des Hinweises auf die Patenterteilung: **12.03.2008**
(21) Anmeldenummer: 01118889.3
(22) Anmeldetag: 17.08.2001
(51) Int. Cl.: A61K 6/083

(54) **Dentalmaterial enthaltend eine sich bei Wärmeanwendung zusammenziehende Komponente**
Dental material containing a heat shrinkable component
Matériau dentaire contenant un composant rétractable à la chaleur

(30) Priorität: 21.08.2000 DE 10040772
(43) Veröffentlichungstag der Anmeldung: 27.02.2002
(73) Patentinhaber: Ivoclar Vivadent AG, 9494 Schaan (LI)
(72) Erfinder: Zanghellini, Gerhard, 9494 Schaan (LI); Hagenbuch, Konrad, 9465 Salez SG (CH)
(74) Vertreter: UEXKÜLL & STOLBERG

(56) Entgegenhaltungen:
- EP-A- 0 872 218
- DE-A- 4 238 470
- US-A- 4 909 736
- US-A- 5 089 306
- US-A- 5 328 372

## Beschreibung

Die Erfindung betrifft Dentalmaterialien, die neben Füllstoff und polymerisierbarer Matrix eine sich bei Wärmebehandlung zusammenziehende Komponente enthalten.

Dentalmaterialien auf der Basis von Kunststoffen enthalten im allgemeinen neben einer polymerisierbaren Matrix einen oder mehrere Füllstoffe. Die Füllstoffe dienen zur Verringerung des Polymerisationsschrumpfes und zur Verbesserung der mechanischen Eigenschaften der gehärteten Materialien. Faserverbundwerkstoffe enthalten faserförmige Füllstoffe, wie Fasermatten oder Faserbündeln, die mit der organischen Polymermatrix getränkt sind. Aus Stabilitätsgründen ist es vorteilhaft, den Füllstoffgehalt möglichst hoch zu wählen. Dies hat jedoch den Nachteil, daß sich die Steifigkeit des ungehärteten Materials erhöht und damit dessen Formgebung erschwert wird.

Die WO95/08300 offenbart ein Verfahren zur Herstellung von Dentalrestaurationen aus Faserverbundwerkstoffen bei dem mit Polymermatrix getränkte Fasermatten auf ein Zahnmodell aufgebracht, in einem Tiefziehverfahren an das Modell angepaßt und anschließend gehärtet werden. Das hierbei erhaltene Gerüst kann z.B. durch das Aufbringen von Verblendmaterial weiterverarbeitet werden.

Die US 5,089,306 offenbart ein Verfahren zum Aufbringen von Glanzmaterialien auf Detalrestaurationen. Die Glanzmaterialien sind auf einen wärmeschrumpfbaren Träger aufgebracht, der sich beim Erhitzen zusammenzieht und dabei das Glanzmaterial auf die Oberfläche der Restauration preßt. Der Träger wird anschließend durch Abbrennen oder Abdampfen entfernt.

Die EP 0 872 218 A2 offenbart ein Verfahren zur Herstellung faserverstärkter Dentalrestaurationen, bei dem der Fasergehalt des ungehärteten Ausgangsmaterials beim Tiefziehen durch das Abpressen überschüssigen Matrixmaterials erhöht wird. Das Verfahren macht die Verwendung besonders gestalteter Gießformen erforderlich.

Die bekannten Tiefziehverfahren weisen den Nachteil auf, daß die Kompressionskräfte nicht homogen von allen Seiten gleichzeitig auf den Werkstoff einwirken und keine gleichmäßige Verdichtung des Materials erzielt wird.

Aufgabe der Erfindung ist die Bereitstellung von Dentalmaterialien, die ohne besondere Hilfsmittel gleichmäßig verdichtet werden können.

Diese Aufgabe wird durch Dentalmaterialien gelöst, die neben Füllstoff und polymerisierbarem Matrix eine sich bei Wärmebehandlung zusammenziehende Komponente enthalten. Diese Komponente wird im folgenden auch als Schrumpfmaterial bezeichnet.

Bevorzugte sich bei Wärmebehandlung zusammenziehende Komponenten sind sogenannte Schrumpffolien und Schrumpfschläuche, d.h.

Kunststoffolien oder Kunststoffschläuche, die sich beim Erwärmen zusammenziehen. Hierbei handelt es sich meist um thermoplastische Kunststoffe, die im Verlauf ihrer Herstellung ein- oder zweidimensional gereckt werden und sich dann beim Erhitzen wieder zusammenziehen.

Geeignete Schrumpffolien und Schrumpfschläuche sind kommerziell erhältlich. Zum Herstellung von Dentalmaterialien haben sich insbesondere Schrumpfmaterialien auf der Basis von Polytetrafluorethylen (PTFE), Polyvinylidenfluorid, wie Vinylidenfluorid-Hexafluorpropylen, Polyolefin, wie Polyethylen und strahlenvernetzte Polyolefine, Polyethylenterephthalat (PETP), und Polyvinylchlorid (PVC) bewährt. Bevorzugt sind einschichtige Materialien.

Die Schrumpftemperatur dieser Materialien liegt üblicherweise im Bereich von 80 bis 330°C. Bevorzugt sind Materialien, die sich im Temperaturbereich von 80 bis 160°C zusammenziehen.

Erfindungsgemäß bevorzugte Materialien weisen eine Schrumpfrate im Bereich von 1,3:1 bis 5:1 auf. Besonders bevorzugt sind Materialien mit einer Schrumpfrate von 3,5:1 bis 4,5:1 und insbesondere etwa 4:1. Schrumpfraten werden, wenn nicht anders angegeben, bei einer Temperatur von 150°C gemessen.

Bevorzugt sind Materialien, die nur eindimensional schrumpfen. Im Fall von Schläuchen findet die Schrumpfung vorzugsweise in radialer Richtung statt während die Schlauchlänge im wesentlichen unverändert bleibt. Die Schrumpfrate in radialer Richtung gibt das Verhältnis des Innendurchmessers des nicht geschrumpften Schlauches zum Innendurchmesser des geschrumpften Schlauches an. Bei einer Schrumpfrate von z.B. 4 ist der Innendurchmesser vor der Schrumpfung viermal größer als danach.

Weiterhin sind transparente Schrumpfmaterialien bevorzugt, da diese eine photochemische Härtung des polymerisierbaren Materials gestatten und zudem die Herstellung natürlich erscheinender Dentalrestaurationen erlauben.

Als polymerisierbare Matrix eignen sich polymerisierbare Monomere, Oligomere, Polymere und Präpolymere sowie Mischungen dieser Stoffe. Bevorzugte Monomere sind ionisch und/oder radikalisch polymerisierbare mono- oder multifunktionelle Monomeren, insbesondere Mono(meth)acrylate, wie Methyl-, Ethyl-, Butyl-, Benzyl-, Furfuryl- oder Phenyl(meth)acrylat, mehrfunktionelle Acrylate und Methacrylate wie zum Beispiel Bisphenol-(A)-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- und Tetraethylenglykoldi(meth)acrylat, Decandioldi(meth)acrylat, Trimethylolpropantri(meth)acrylat, Pentaerythrittetra(meth)acrylat und Butandioldi(meth)acrylat, 1,10-Decandioldi(meth)acrylat oder 1,12-Dodecandiol-di(meth)acrylat.

Unter den aromatischen Dimethacrylatharzen sind Bisphenol-A-derivate wie Bisphenol-A-glycidyl-dimethacrylat (bis-GMA), Urethanmethacrylat (UDMA), Triethylenglycoldimethacrylat (TEDMA) und Mischungen davon bevorzugt.

Weiter bevorzugte polymerisierbare Materialien sind Polycarbonat-di(meth)acrylate, insbesondere das Kondensationsprodukt aus einem Hydroxyalkylmethacrylat, vorzugsweise 2-Hydroxyethylmethacrylat, und einem Bis(chlorformiat), vorzugsweise Triethylenglykolbis(chlorformiat)), Polycarbonat-tri- oder -tetra-(meth)-acrylate, Urethan-di-, tri-, tetra-(meth)acrylate und Mischungen davon. Monomere dieses Typs werden in der DE 36 32 868 A1 und der US 5,444,104 beschrieben.

Erfindungsgemäß kann vorzugsweise auch Harz auf der Basis von Bis-GMA verwendet werden, das durch Copolymerisation mit Materialien mit geringem Molekulargewicht modifiziert wurde, wie Bisphenolglycidyldimethacrylat (BIS-MA), Bisphenolethylmethacrylat (BIS-EMA), Bisphenolpropylmethacrylat (BIS-PMA), Ethylenglycoldimethacrylat (EGDMA), Diethylenglycoldimethacrylat (DEGDMA), Triethylenglycoldimethacrylat (TEGDMA), Triethylenglycolmethacrylat (TEGMA), Methylmethacrylat (MMA), und Polyurethanfluormethacrylat (PFUMA).

Besonders bevorzugt ist eine polymerisierbare Matrix auf der Basis einer Mischung von Bis-GMA, Decandioldimethacrylat (DDDMA), Triethylenglycoldimethacrylat (TEGDMA) und Urethandimethacrylat (UDMA). Eine besonders bevorzugte polymerisierbare Matrix enthält 65 bis 75 Gew.- % Bis-GMA, 10 bis 20 Gew.-% Triethylenglycoldimethacrylat und 5 bis 15 Gew.-% hochdisperses Siliciumdioxid (bezogen auf die Masse der Matrix ohne faserförmige Füllstoffe und ohne Schrumpfmaterial).

Zur Initiierung der radikalischen Polymerisation enthält die Matrix vorzugsweise thermische und/oder Photoinitiatoren.

Bevorzugte Initiatoren für die thermische Härtung sind Peroxide, wie beispielsweise Dibenzoylperoxid, Dilaurylperoxid, tert.-Butylperoctoat und tert.-Butylperbenzoat sowie Azobisisobutyroethylester, Benzpinakol und 2,2-Dimethylbenzpinakol.

Bevorzugte Photoinitiatoren sind Benzophenon und Benzoin sowie deren Derivate, α-Diketone und deren Derivate, wie beispielsweise 9,10-Phenanthrenchinon, Diacetyl und 4,4-Dichlorbenzil. Besonders bevorzugte Photoinitiatoren sind Champherchinon und 2,2-Methoxy-2-phenyl-acetophenon und insbesondere Kombinationen von α-Diketonen mit Aminen als Reduktionsmittel, wie zum Beispiel N-Cyanoethyl-N-methylanilin, 4-(N,N-Dimethylamino)-benzoesäureester, N,N-Dimethylaminoethylmethacrylat, N,N-Dimethyl-sym.-xylidin oder Triethanolamin. Darüber hinaus sind Acylphosphine, wie zum Beispiel 2,4,6-Trimethylbenzoyldiphenyl- oder Bis-(2,6-dichlorbenzoyl)-4-N-propylphenylphosphinoxid, als Photoinitiatoren geeignet.

Als Füllstoffe sind organische und anorganische faserförmige Materialien, insbesondere Fasermatten und/oder uniaxial ausgerichtete Faserbündel bevorzugt. Die Faserbündel können zur weiteren Steigerung der mechanischen Festigkeit mit Fasern umwikkelt oder umflochten sein. Das Material kann ein oder mehrere Faserbündel enthalten.

Bevorzugte Fasern sind Glasfasern, Aramidfasern, Kohlenstoffasern und Polyethylenfasern (PE-Fasern) und deren Kombinationen.
Bevorzugte partikuläre Füllstoffe sind amorphe kugelförmige Materialien auf der Basis von Mischoxiden aus SiO₂, ZrO₂ und/oder TiO₂ (DE 40 29 230 A1), mikrofeine Füllstoffe, wie pyrogene Kieselsäure oder Fällungskieselsäure sowie Makro- (Partikelgröße von 5 µm bis 200 µm) oder Minifüllstoffe (Partikelgröße von 0,5 bis 5 µm), wie Quarz-, Glaskeramik- oder Glaspulver mit einer durchschnittlichen Teilchengröße von 0,5 µm bis 5 µm sowie röntgenopake Füllstoffe, wie Ytterbiumtrifluorid.

Kombinationen von faserförmigen und partikulären Füllstoffen sind möglich.

Die Mischungen können darüber hinaus weitere Additive wie Färbemittel (Pigmente und Farbstoffe), Stabilisatoren, Aromastoffe, mikrobiozide Wirkstoffe, Weichmacher und/oder UV-Absorber enthalten.

Geeignete Faserverbundwerkstoffe, d.h. Kombinationen von organischer Matrix, faserförmigem Füllstoff, gegebenenfalls partikulärem Füllstoff, Hilfsstoffen und Additiven und werden in der WO95/08300 beschrieben.

Bevorzugte Faserverbundwerkstoffe zur Verwendung mit Schrumpfmaterialien enthalten 7 bis 94 Gew.-% faserförmigen Füllstoff und 6 bis 93 Gew.-% polymerisierbare Matrix. Besonders bevorzugt sind Werkstoffe die 28 bis 82 Gew.-% und insbesondere 35 bis 65 Gew.-% faserförmigen Füllstoff enthalten.

Der Anteil nicht-faserförmiger Füllstoffe liegt vorzugsweise im Bereich von 0 bis 30 Gew.-%, insbesondere 2 bis 15 Gew.-% und besonders bevorzugt im Bereich von 3,5 bis 5,5 Gew.-%. Gemäß einer ganz besonders bevorzugten Ausführungsform enthält das Material 3,5 bis 5,5 Gew.-% hoch disperse Kieselsäure als zusätzlichen Füllstoff.

Initiatoren und ggf. Beschleuniger werden vorzugsweise in einer Menge von jeweils 0,01 bis 3,0 Gew-%, besonders bevorzugt in einer Menge von jeweils 0,05 bis 1,0 Gew.-% bezogen auf die Masse an Matrixmaterial verwendet. Die Gesamtmenge an Katalysatoren und Stabilisatoren beträgt vorzugsweise jeweils 0,3 bis 0,5 Gew.-% basierend auf der Gesamtmasse des Faserverbundwerkstoffs.

Andere Additive, wie z.B. Pigmente, werden üblicherweise in einer Menge von weniger als 0,1 Gew.-% eingesetzt.

Faserförmige und nicht-faserförmige anorganische Füllstoffe werden vor dem Einarbeiten in die Matrix vorzugsweise hydrophobisiert, besonders bevorzugt silanisiert, d.h. mit einer organischen Siliciumverbindung wie einem Aryloxysilan, Alkoxysilan und/oder einem Halogensilan wie (Meth)acryloylalkoxysilan behandelt.

Alle oben und im folgenden genannten Prozentangaben beziehen sich, wenn nicht anders angegeben, auf die Masse des Faserverbundwerkstoffs ohne die Schrumpfkomponente.

Die Zusammensetzung von besonders bevorzugten Faserverbundwerkstoffen ist im folgenden beispielhaft angegeben (alle Angaben sind in Gew.-%):

| Komponente | Werkstoff Nr. 1 | Werkstoff Nr. 2 | Werkstoff Nr. 3 |
|---|---|---|---|
| Bis-GMA | 38.7 | 24.6 | 35.3 |
| DDDMA | 0.5 | 0.3 | 0.4 |
| TEDMA | 9.7 | 6.2 | 8.8 |
| Hoch disperse Kieselsäure | 5.5 | 3.5 | 5.0 |
| Katalysatoren und Stabilisatoren | ≤0.5 | ≤0.3 | ≤0.4 |
| Pigmente | ≤0.1 | ≤0.1 | ≤0.1 |
| Glasfasern | 45.0 | 65.0 | 50.0 |

Gemäß einer bevorzugten Ausführungsform hat die sich bei Wärmeeinwirkung zusammenziehende Komponente die Form eines Schlauches. Dieser wird mit Füllstoff, vorzugsweise einem faserförmigem Füllstoff, und Matrix gefüllt. Beim Erwärmen des Schlauches verringert sich dessen innerer und äußerer Durchmesser, wodurch die Fasern in der Matrix gleichmäßig verdichtet werden und überschüssiges Matrixmaterial an den Schlauchenden aus dem Schlauch herausgepreßt wird. Bei langen Schläuchen kann die Verwendung perforierter Schläuche vorteilhaft sein, um das Entweichen des Matrixmaterials zu erleichtern. Diese Verdichtung führt zu einer Erhöhung des Fasergehalts und damit der Festigkeit des Dentalwerkstoffs.

Im allgemeinen läßt sich der Fasergehalt um das ein- bis vierfache erhöhen. Das Maß der Verdichtung läßt sich über das Schrumpfverhältnis des Schrumpfmaterials so steuern, daß die gewünschten mechanischen Eigenschaften erzielt werden.

Zur Bestimmung des Verdichtungsverhältnisses fertigt man Querschnitte von gehärtetem Verbundwerkstoff mit und ohne Schrumpfmaterial an und ermittelt jeweils das Verhältnis der Flächen, die von Fasern und Matrixmaterial eingenommen werden. Die Verdichtung entspricht dem Quotienten aus dem Flächenverhältnis von Faser zu Matrix mit Schrumpfmaterial und dem Flächenverhältnis von Faser zu Matrix ohne Schrumpfmaterial. Werden nur anorganische Fasern, wie z.B. Glasfasern, eingesetzt, kann die Verdichtung bzw. der Fasergehalt auch über den Glührückstand ermittelt werden.

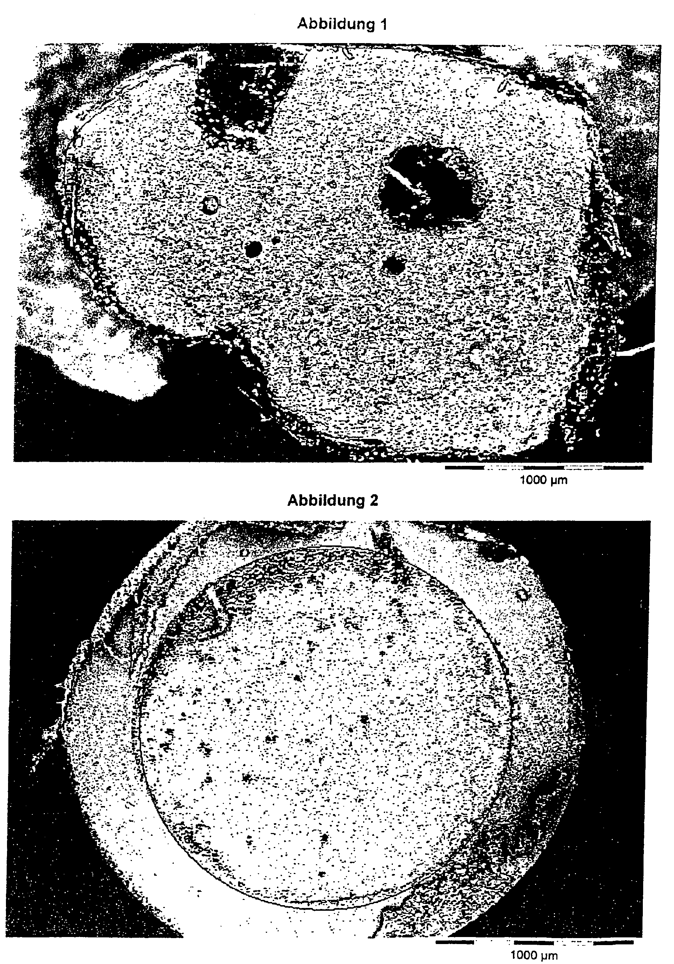

Abbildung 1 zeigt eine elektronenmikroskopische Aufnahme eines Querschnitts durch einen stäbchenförmigen Verbundwerkstoff, der ohne Schrumpfmaterial gehärtet wurde, Abbildung 2 die Aufnahme eines Querschnitts durch einen zweiten Verbundwerkstoff, der in einem Schrumpfschlauch enthalten ist. Der Schnitt wurde in beiden Fällen senkrecht zur Faserrichtung und zur Längsachse des Materials durchgeführt. Die Zusammensetzungen der Verbundwerkstoffe vor der Härtung und Verdichtung waren identisch und entsprechen in beiden Fällen dem oben beschriebenen Werkstoff Nr. 2.

Bereits ein visueller Vergleich der Abbildungen läßt erkennen, daß die Faserdichte bei dem Material mit Schrumpfschlauch deutlich höher ist als bei dem Material ohne Schrumpfmaterial. Letzteres weist zudem große Lufteinschlüsse auf.

Eine Messung der Querschnittsflächen ohne und mit Schrumpfschlauch ergab Werte von 4,44 mm² (ohne Berücksichtigung des großen Lufteinschlusses) und 2,81 mm².

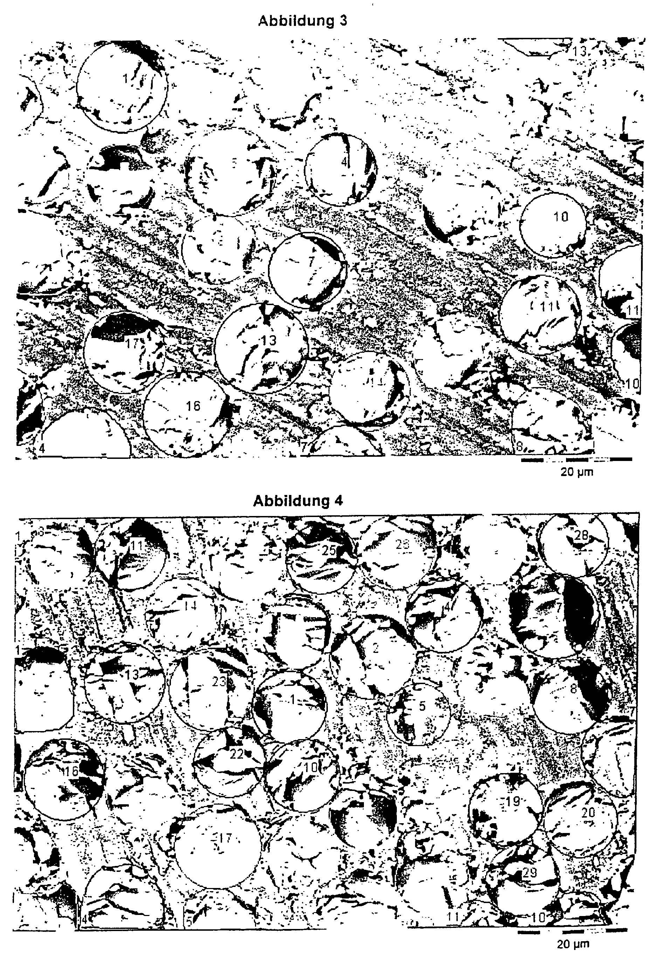

Die Abbildungen 3 und 4 zeigen Ausschnitte derselben Querschnitte bei einer stärkeren Vergrößerung. Es kann deutlich zwischen Fasern und Matrix unterschieden werden. In Abbildung 3 (Material ohne Schrumpfschlauch) beträgt die von den Fasern belegte Fläche 3863 µm², die Fläche der Matrix 4573 µm² und die Gesamtfläche 8436 µm². Das Flächenverhältnis von Fasern zu Matrix ist dementsprechend 0,84.

In Abbildung 4 (Material mit Schrumpfschlauch) beträgt die von den Fasern belegte Fläche 5385 µm², die Fläche der Matrix 3237 µm² und die Gesamtfläche 8622 µm². Das Flächenverhältnis von Fasern zu Matrix ist 1,66. Damit ergibt sich eine Verdichtung von 1,66 : 0,84 = 1,98.

Gemäß einer weiter bevorzugten Ausführungsform werden uniaxial ausgerichtete Fasern mit einem Schlauch aus Fasergewebe oder -gestrick ummantelt und diese Kombination dann mit einer sich bei Wärmebehandlung zusammenziehenden Komponente umgeben. Die beim Erwärmen einsetzende Verdichtung wird die Orientierung der Fasern und damit die Querfestigkeit, Druckfestigkeit und Torsionsfestigkeit des Stabes verbessert.

Schlauch- oder stabförmigen Komponenten eignen sich insbesondere zur Herstellung von faserverstärkten dentalen Brückengerüsten. Die räumlichen Abmessungen der Komponenten sind auf die Anfertigung von Dentalrestaurationen abgestimmt. Bevorzugt sind stabförmige Materialien mit einer Länge von 10 bis 160 mm und einem Durchmesser von 2 bis 4 mm (gemessen ohne Schrumpfmaterial). Die Materialien weisen vorzugsweise einen runden Querschnitt auf. Sie zeichnen sich dadurch aus, daß das mit organischer Matrix imprägnierte Faserbündel von außen trocken und damit von Hand ohne Verschmutzungsgefahr und ohne Gefahr, die Faserbündel zu kontaminieren, leicht zu verarbeiten ist.

Die erfindungsgemäßen Materialien werden beispielsweise unter Verwendung von Modellen, wie Sägeschnittmodellen aus Hartgips, geformt und anschließend erwärmt. Hierbei zieht sich die bei Wärmebehandlung zusammenziehende Komponente zusammen und bewirkt so eine Verdichtung des Dentalmaterials. Anschließend wird das Material gehärtet, vorzugsweise durch radikalische Polymerisation.

Das Schrumpfmaterial wird nach der Polymerisation vorzugsweise entfernt und der verbleibende, gehärtete Verbundwerkstoff weiter verarbeitet, beispielsweise mit geeigneten Materialien verblendet.

Es ist jedoch auch denkbar, daß das Schrumpfmaterial bei der Polymerisation über polymerisierbare Gruppen fest mit der Matrix verbunden wird.

Die Verdichtung von Faserverbundwerkstoffen läßt sich auch durch die Verwendung von Schrumpfbändern erreichen. Der Faserverbundwerkstoff wird, ggf. zusammen mit dem Modell und vorzugsweise nach dem Formen, mit einem Band aus dem sich bei Wärmebehandlung zusammenziehenden Material umwickelt und anschließend erwärmt. Hierbei zieht sich das Band zusammen und verdichtet das Wickelgut. Durch die Verwendung von Schrumpfbändern lassen sich auch komplizierte Konstruktionen homogen verdichten. Nach der Polymerisation der Matrix, die während oder im Anschluß an das Erwärmen erfolgen kann, werden die Bänder von der Dentalrestauration entfernt und diese gegebenenfalls weiterverarbeitet, beispielsweise verblendet oder überkront. Bei Schrumpfbändern dienen die Überlappungsstellen der Bänder als Austrittsöffnungen für das Monomer.

Die Bänder haben vorzugsweise eine Breite von 5 bis 100 mm, besonders bevorzugt 10 bis 30 mm. Die Dicke der Bänder ist variabel und beträgt typischerweise etwa 0,4 mm. Die Schrumpfrate der Bänder, gemessen in Längsrichtung, beträgt vorzugsweise 1,3:1 bis 4:1.

Für die Weiterverarbeitung der gehärteten Materialien, z.B. für das Aufbringen von Verblendmaterialien, ist es im allgemeinen erforderlich, die Oberfläche des Faserverbundwerkstoffs anzurauhen. Dieser Schritt ist mit zusätzlichem Aufwand verbunden. Aus diesem Grunde wird vor dem Schrumpfmaterial vorzugsweise zuerst ein sogenanntes Abreißgewebe auf die Dentalrestauration aufgebracht. Als Abreißgewebe werden solche Materialien bezeichnet, die nach der Polymerisation von dem gehärteten Faserverbundwerkstoff entfernt (abgerissen) werden können und dabei eine saubere, rauhe Oberfläche zurücklassen. Der Faserverbundwerkstoff kann nach dem Entfernen das Abreißgewebes ohne zusätzliches Aufrauhen der Oberfläche weiterverarbeitet werden.

Gemäß einer weiteren Ausführungsform der Erfindung werden Kappen aus Schrumpfmaterial mit polymerisierbarem Kompositmaterial gefüllt und dann durch Schrumpfung an ein Modell, wie zum Beispiel einen Zahnstumpf, angepaßt. Größe und Form der Kappen sind an die Verwendung mit Zähnen und Zahnstümpfen abgestimmt. Anschließend wird das polymerisierbare Material gehärtet, ggf. das Schrumpfmaterial entfernt und der gehärtete Formkörper ggf. weiterverarbeitet, beispielsweise durch das Aufbringen von Verblendmaterial. Auf diese Weise lassen sich z.B. dentale Kronen und Brückenpfeiler für die permanente und temporäre Anwendung herstellen.

Im Folgenden wird die Herstellung einer Krone genauer beschrieben. Der zu restaurierende Zahn wird auf an sich bekannte Weise zu einem Stumpf beschliffen. Von diesem Stumpf wird mit Abformmaterialien, wie beispielsweise Alginat, Polyether oder Silikon, eine Negativform angefertigt, die anschließend mit einem Modellmaterial, wie Gips oder Epoxyharz, ausgegossen wird. Auf diese Weise wird ein Positivmodell erhalten, das z.B. mit einer Alginatlösung (im Fall eines Gipsmodells) oder einer Wachslösung (im Fall eines Epoxymodells) isoliert wird. Auf den isolierten Modellstumpf wird dann ein Käppchen aus einem Schrumpfmaterial aufgebracht, das mit einem polymerisierbaren Material gefüllt ist. Das Käppchen wird so auf den Stumpf aufgesetzt, daß es diesen umhüllt. Durch Erhitzen wird das Käppchen geschrumpft und dabei das polymerisierbare Material an den Stumpf angepaßt und gleichzeitig verdichtet. Anschließend wird das Material durch Licht oder weiteres Erhitzen ausgehärtet und das gehärtete Material nach üblichen zahntechnischen Methoden zur fertigen Krone ausgearbeitet, z.B. durch Aufbringen eines geeigneten Verblendwerkstoffs. Hierzu wird die Oberfläche des Kronenrohlings ggf. angerauht und das Verblendmaterial auf die rauhe Oberfläche aufgetragen. Der Auftrag erfolgt in der Regel in zwei oder mehr Schichten, wobei die einzelnen Schichten nach dem Auftrag jeweils gehärtet werden. Nach dem Aufbringen der letzten Schicht wird die Krone endgehärtet, poliert, gereinigt und vom Zahnarzt dem Patienten eingegliedert.

Zur Herstellung einer Brücke werden in die beiden der Zahnlücke benachbarten Zähne vom Zahnarzt mit geeigneten Instrumenten distal bzw. mesial auf an sich bekannte Weise Füllungskavitäten präpariert und dann von der Mundsituation wie oben beschrieben ein Positivmodell angefertigt und dieses isoliert. Die Isolierung verhindert ein Verkleben des Gerüstmaterials mit dem Modell. Anschließend wird z.B. ein stäbchenförmiges, faserverstärktes Verbundmaterial, das vorzugsweise in einem Schrumpfschlauch enthalten ist, auf eine Länge zugeschnitten, die dem Abstand der beiden präparierten Kavitäten entspricht. Dieser Strang wird dann in die Kavitäten so eingelegt, daß er die Zahnlücke überspannt. Damit sich der Strang während des Erhitzens nicht verformt, kann er an beiden Enden fixiert werden, z.B. indem dort austretendes Matrixmonomer durch kurzzeitiges Bestrahlen mit einem Lichtleiter gehärtete wird. Alternativ oder gleichzeitig kann der Strang mit Silikonmaterial so abgestützt werden, daß er sich nicht nach unten durchbiegen kann. Ein Abstützen ist vor allem dann erforderlich, wenn große Lücken (größer als etwa 6 mm) überspannt werden müssen. Danach wird das Material mit einer Wärmequelle, z.B. mit einem Warmluftgebläse oder einem IR-Strahler, auf die Schrumpftemperatur erwärmt. Nach der Schrumpfung wird der Strang mit einer Lichtquelle, z.B. Heliolux oder Targis Power, ausgehärtet, der Schrumpfschlauch entfernt und das Gerüst mit einem Verblendmaterial auf übliche Weise verblendet. Zum Entfernen des Schrumpfschlauchs wird dieser vorzugsweise aufgeschnitten und dann abgeschält.

## Patentansprüche

1. Dentalmaterial, enthaltend Füllstoff und eine polymerisierbare Matrix, **dadurch gekennzeichnet, daß** es weiterhin eine sich bei Wärmebehandlung zusammenziehende Komponente enthält.

2. Dentalmaterial nach Anspruch 1, **dadurch gekennzeichnet, daß** es faserförmigen Füllstoff enthält.

3. Dentalmaterial nach Anspruch 2, **dadurch gekennzeichnet, daß** es Glasfasern, Aramidfasern, Kohlenstoffasern und/oder Polyethylenfasern enthält.

4. Dentalmaterial nach einem der Ansprüche 1 bis 3, **dadurch gekennzeichnet, daß** die sich bei Wärmebehandlung zusammenziehende Komponente nach der Polymerisation der Matrix entfernbar ist.

5. Dentalmaterial nach einem der Ansprüche 1 bis 4, **dadurch gekennzeichnet, daß** die sich bei Wärmebehandlung zusammenziehende Komponente eine Schrumpfrate von 1,3:1 bis 5:1 aufweist.

6. Dentalmaterial nach einem der Ansprüche 1 bis 5, **dadurch gekennzeichnet, daß** die sich bei Wärmebehandlung zusammenziehende Komponente transparent ist.

7. Dentalmaterial nach einem der Ansprüche 1 bis 6, **dadurch gekennzeichnet, daß** die sich bei Wärmebehandlung zusammenziehende Komponente die Form eines Schlauches hat, der mit Füllstoff und einem polymerisierbaren Matrixmaterial gefüllt ist.

8. Dentalmaterial nach einem der Ansprüche 2 bis 7, **dadurch gekennzeichnet, daß** es als faserförmigen Füllstoff ein oder mehrere uniaxial ausgerichtete Faserbündel enthält.

9. Dentalmaterial nach einem der Ansprüche 1 bis 6, **dadurch gekennzeichnet, daß** die sich bei Wärmebehandlung zusammenziehende Komponente die Form einer Kappe hat, die mit Füllstoff und einem polymerisierbaren Matrixmaterial gefüllt ist.

10. Dentalmaterial nach einem der Ansprüche 1 bis 6, **dadurch gekennzeichnet, daß** die sich bei Wärmebehandlung zusammenziehende Komponente die Form einer Folie oder eines Bands hat.

11. Dentalmaterial nach einem der Ansprüche 1 bis 10, **dadurch gekennzeichnet, daß** es 7 bis 94 Gew.-% faserförmigen Füllstoff und 6 bis 93 Gew.-% polymerisierbare Matrix enthält, bezogen auf die Masse des Faserverbundwerkstoffs ohne die sich bei Wärmebehandlung zusammenziehende Komponente.

12. Dentalmaterial nach einem der Ansprüche 1 bis 11, **dadurch gekennzeichnet, daß** es 0 bis 30 Gew.-% nicht-faserförmige Füllstoffe enthält, bezogen auf die Masse des Faserverbundwerkstoffs ohne die sich bei Wärmebehandlung zusammenziehende Komponente.

13. Dentalmaterial nach einem der Ansprüche 1 bis 12, **dadurch gekennzeichnet, daß** es als polymerisierbare Matrix eine Mischung von Bis-GMA, Decandioldimethacrylat (DDDMA), Triethylenglycoldimethacrylat (TEGDMA) und Urethandimethacrylat (UDMA) enthält.

14. Dentalmaterial nach Anspruch 13, **dadurch gekennzeichnet, daß** die polymerisierbare Matrix 65 bis 75 Gew.-% Bis-GMA, 10 bis 20 Gew.-% Triethylenglycoldimethacrylat und 5 bis 15 Gew.-% hochdisperses Siliciumdioxid enthält.

15. Verfahren zur Herstellung von Dentalrestaurationen, bei dem man ein Füllstoff und polymerisierbare Matrix enthaltendes Dentalmaterial auf ein Modell aufbringt, man das Modell dann mit einem sich bei Wärmebehandlung zusammenziehenden Material bedeckt und man das Modell anschließend einer Wärmebehandlung aussetzt.

16. Verfahren zur Herstellung von Dentalrestaurationen, bei dem man eine Füllstoff und polymerisierbare Matrix enthaltende Kappe aus einem sich bei Wärmebehandlung zusammenziehenden Material auf ein Zahnmodell aufbringt und man das Modell dann einer Wärmebehandlung aussetzt.

17. Verwendung eines sich bei Wärmebehandlung zusammenziehenden Materials zur Herstellung von Dentalmaterialien und/oder Dentalrestaurationen.

## Claims

1. Dental material comprising filler and a polymerisable matrix, **characterised in that** it further comprises a component which contracts during thermal treatment.

2. Dental material according to claim 1, **characterised in that** it comprises fibrous filler.

3. Dental material according to claim 2, **characterised in that** it comprises glass fibres, aramid fibres, carbon fibres and/or polyethylene fibres.

4. Dental material according to one of claims 1 to 3, **characterised in that** the component which contracts during thermal treatment can be removed after polymerisation of the matrix.

5. Dental material according to one of claims 1 to 4, **characterised in that** the component that contracts during thermal treatment has a shrinkage rate of 1.3: 1 to 5: 1.

6. Dental material according to one of claims 1 to 5, **characterised in that** the component that contracts during thermal treatment is transparent.

7. Dental material according to one of claims 1 to 6, **characterised in that** the component which contracts during thermal treatment is shaped like a tube which is filled with filler and a polymerisable matrix material.

8. Dental material according to one of claims 2 to 7, **characterised in that** it comprises one or a plurality of uniaxially oriented fibre bundles as the fibrous filler.

9. Dental material according to one of claims 1 to 6, **characterised in that** the component which contracts during thermal treatment is shaped like a cap which is filled with filler and a polymerisable matrix material.

10. Dental material according to one of claims 1 to 6, **characterised in that** the component that contracts during thermal treatment is shaped like a film or a strip.

11. Dental material according to one of claims 1 to 10, **characterised in that** it comprises 7 to 94 wt.-% of fibrous filler and 6 to 93 wt.-% of polymerisable matrix relative to the mass of the fibre composite without the component which contracts during thermal treatment

12. Dental material according to one of claims 1 to 11, **characterised in that** it comprises 0 to 30 wt.-% of non-fibrous fillers relative to the mass of the fibre composite without the component which contracts during thermal treatment.

13. Dental material according to one of claims 1 to 12, **characterised in that** it comprises, as the polymerisable matrix, a mixture of bis-GMA, decane diol dimethacrylate (DDDMA), triethylene glycol dimethacrylate (TEGDMA) and urethane dimethacrylate (UDMA).

14. Dental material according to claim 13, **characterised in that** the polymerisable matrix comprises 65 to 75 wt.% bis-GMA, 10 to 20 wt.% triethylene glycol dimethacrylate and 5 to 15 wt.% of highly-dispersed silicon dioxide.

15. Process for manufacturing dental restorations, in which a dental material comprising a filler and a polymerisable matrix is applied to a cast, the cast is then covered with a material which contracts during thermal treatment and the cast is subsequently subjected to a thermal treatment.

16. Process for manufacturing dental restorations, in which a cap, made from a material which contracts during thermal treatment, comprising a filler and polymerisable matrix, is applied to a tooth cast and the cast is then subjected to a thermal treatment.

17. Use of a material which contracts during thermal treatment for manufacturing dental materials and/or dental restorations.

## Revendications

1. Matériau dentaire, contenant une charge et une matrice polymérisable, **caractérisé en ce qu'**il contient en outre un composant qui se contracte lors du traitement thermique.

2. Matériau dentaire selon la revendication 1, **caractérisé en ce qu'**il contient une charge sous forme fibreuse.

3. Matériau dentaire selon la revendication 2, **caractérisé en ce qu'**il contient des fibres de verre, des fibres aramides, des fibres de carbone et/ou des fibres de polyéthylène.

4. Matériau dentaire selon l'une quelconque des revendications 1 à 3, **caractérisé en ce que** le composant qui se contracte lors du traitement thermique peut être retiré suite à la polymérisation de la matrice.

5. Matériau dentaire selon l'une quelconque des revendications 1 à 4, **caractérisé en ce que** le composant qui se contracte lors du traitement thermique a un taux de rétraction qui va de 1,3/1 à 5/1.

6. Matériau dentaire selon l'une quelconque des revendications 1 à 5, **caractérisé en ce que** le composant qui se contracte lors du traitement thermique est transparent.

7. Matériau dentaire selon l'une quelconque des revendications 1 à 6, **caractérisé en ce que** le composant qui se contracte lors du traitement thermique a la forme d'un tube, qui est rempli d'une charge et d'un matériau de matrice polymérisable.

8. Matériau dentaire selon l'une quelconque des revendications 2 à 7, **caractérisé en ce qu'**il contient, comme charge sous forme fibreuse, un ou plusieurs faisceaux de fibres agencés de manière uniaxiale.

9. Matériau dentaire selon l'une quelconque des revendications 1 à 6, **caractérisé en ce que** le composant qui se contracte lors du traitement thermique a la forme d'une coiffe, qui est remplie d'une charge et d'un matériau de matrice polymérisable.

10. Matériau dentaire selon l'une quelconque des revendications 1 à 6, **caractérisé en ce que** le composant qui se contracte lors du traitement thermique a la forme d'une pellicule ou d'une bande.

11. Matériau dentaire selon l'une quelconque des revendications 1 à 10, **caractérisé en ce qu'**il contient 7 à 94 % en poids de charge sous forme fibreuse et 6 à 93 % en poids de matrice polymérisable, par rapport à la masse du matériau composite renforcé par des fibres sans le composant qui se contracte lors du traitement thermique.

12. Matériau dentaire selon l'une quelconque des revendications 1 à 11, **caractérisé en ce qu'**il contient 0 à 30 % en poids de charges qui ne sont pas sous forme fibreuse, par rapport à la masse du matériau composite renforcé par des fibres sans le composant qui se contracte lors du traitement thermique.

13. Matériau dentaire selon l'une quelconque des revendications 1 à 12, **caractérisé en ce que**, comme matrice polymérisable, il contient un mélange de Bis-GMA, diméthacrylate de décanediol (DDDMA), diméthacrylate de triéthylèneglycol (TEGDMA) et diméthacrylate d'uréthanne (UDMA).

14. Matériau dentaire selon la revendication 13, **caractérisé en ce que** la matrice polymérisable contient 65 à 75 % en poids de Bis-GMA, 10 à 20 % en poids de diméthacrylate de triéthylèneglycol et 5 à 15 % en poids d'oxyde de silicium hautement dispersé.

15. Procédé permettant de fabriquer des restaurations dentaires, dans lequel on applique un matériau dentaire contenant une charge et une matrice polymérisable sur un modèle, on recouvre alors le modèle d'un matériau qui se contracte lors du traitement thermique et on soumet alors le modèle à un traitement thermique.

16. Procédé permettant de fabriquer des restaurations dentaires, dans lequel on applique une coiffe contenant une charge et une matrice polymérisable, constituée d'un matériau qui se contracte lors du traitement thermique, sur un matériau dentaire, et on soumet alors le modèle à un traitement thermique.

17. Utilisation d'un matériau qui se contracte lors d'un traitement thermique afin de fabriquer des matériaux dentaires et / ou des restaurations dentaires.
